# EUROPEAN PATENT SPECIFICATION

(11) **EP 3 932 340 B1**
(45) Date of publication and mention of the grant of the patent: **13.03.2024**
(21) Application number: 21181928.9
(22) Date of filing: 28.06.2021
(51) Int. Cl.: A61B 17/22, A61F 2/95, A61F 2/954, A61F 2/958, A61M 25/10, A61M 25/00, A61B 17/00

(54) **ISOLATED STENTING WITH DISTAL BALLOON**
ISOLIERTES STENTING MIT DISTALEM BALLON
POSE D'ENDOPROTHÈSE ISOLÉE AVEC BALLONNET DISTAL

(30) Priority: 29.06.2020 US 202016914663
(43) Date of publication of application: 05.01.2022
(73) Proprietor: Neuravi Limited, Galway H91 K5YD (IE)
(72) Inventor: KEATING, Karl, Galway, H91 K5YD (IE); KELLY, Ronald, Galway, H91 K5YD (IE)
(74) Representative: Carpmaels & Ransford LLP

(56) References cited:
- WO-A1-97/24154
- DE-A1- 19 526 784
- US-A1- 2005 256 565
- US-B1- 6 485 500

## Description

### FIELD OF INVENTION

The present invention generally relates to medical devices for treating intravascular lesions, and more specifically to systems for catheter based treatments of lesions relating to intracranial atherosclerosis disease (ICAD).

### BACKGROUND

Atherosclerosis results from lesions which narrow and reduce the space in the lumen of vessels in the vasculature. Such lesions are usually composed of plaque, which can be fat, cholesterol, calcium, or other components of the blood. Severe occlusion or closure can impede the flow of oxygenated blood to different organs and parts of the body and result in other cardiovascular disorders such as heart attack or stroke. Narrowing of vessels, or stenosis, increases the risk that clots, and other emboli can lodge at such locations, especially in the neurovascular where vessel diameters are already small. ICAD is the narrowing of those arteries and vessels supplying blood to the brain and represents the most common proximate mechanism of ischemic stroke.

Current methods for treating vascular occlusions include utilizing drugs, such as anticoagulants or anti-platelet agents, as well as medical procedures such as surgical endarterectomy, angioplasty, and stenting. Mechanical procedures often involve using medical devices to retrieve an occlusive clot and then utilizing balloons and stents to open a narrowed artery. Following the use of a stentriever or other clot retrieval device, a balloon is delivered to a target site and inflated to dilate the stenosis. The balloon can then be removed and exchanged through a catheter for a stent delivery device. If desirable, once the stent is in place a balloon can be inflated inside the stent to press the struts of the stent scaffold frame firmly against the inner wall of the vessel. Crossing the occlusion with devices such as stentreivers and stents can increase the likelihood that the ICAD ruptures or fragments. Such fragments can include but are not limited to blood clots, plaque, and other thrombi debris. The fragments can lead to vascular occlusions downstream of the lesion, causing extensive stroke or death. DE19526784A1 describes a double balloon catheter consisting of a catheter shaft which carries two balloon sections at its distal end, and connection sections as well as a further connection for a guide wire. The distal balloon is a dilation balloon while the proximal balloon is a stent balloon. The distal balloon can expand more than the stent balloon, and the dilation balloon operating pressure in 6-15 bar while that of the stent balloon is up to 25 bar.

Applicants therefore recognize a need for systems and devices to continue to address and improve treatments for intravascular occlusions, specifically ICAD.

### SUMMARY

It is an object of the present invention to provide catheter based systems for isolated stenting of an intravascular lesion. Expandable occlusion devices can be expanded in a distal direction and a proximal direction in relation to the lesion to occlude vasculature. A stent can be deployed across the lesion while the occlusion devices are in place. Fragments dislodged during stenting can be aspirated. The invention is defined by claim 1. Embodiments of the invention are defined by the dependent claims.

The intravascular treatment system includes a delivery tube, a distal balloon, an angioplasty balloon, a stent, and one or more inflation lumens. The treatment system can also include an expandable element such as a proximal balloon. The treatment system can further include an outer catheter sized to traverse vascular and sized to allow the delivery tube, distal balloon, angioplasty balloon, and stent to traverse therethrough.

The delivery tube can be sized to traverse vasculature.

The expandable element can be disposed on the delivery tube in the proximal direction in relation to the angioplasty balloon. Alternatively, the expandable element can be disposed on the outer catheter. In either case, the expandable element can be a balloon (referred to herein as a "proximal balloon") inflatable by an inflation lumen of the one or more inflation lumens. Alternatively, the expandable element can be self-expanding or expandable by other means as understood by a person of ordinary skill in the art according to the teachings of the present disclosure. The expandable element can be porous.

The distal balloon is disposed on the delivery tube. The distal balloon is disposed near a distal end of the delivery tube. According to the invention, the distal balloon is expandable at a lower pressure than the angioplasty balloon. When both the proximal balloon and the distal balloon are inflated by the same inflation lumen, the distal balloon can be expandable at a different pressure than the angioplasty balloon and/or the proximal balloon.

The angioplasty balloon is disposed on the delivery tube. The angioplasty balloon is disposed in the proximal direction in relation to the distal balloon. The angioplasty balloon can include an expandable non-compliant wrap configured to expand the stent. Additionally, or alternatively, the angioplasty balloon can include a non-compliant membrane, the stent can restrict expansion of the angioplasty balloon during delivery, and the expanded diameter of the angioplasty balloon can be determined by the shape of the non-compliant membrane. In either case, the angioplasty balloon is expandable at a different pressure than the distal balloon and the angioplasty balloon is inflated by the same inflation lumen as the distal balloon.

The stent is disposed over the angioplasty balloon. The stent can be a balloon expandable stent, meaning inflation of the balloon is necessary to expand the stent, i.e. the stent is not self-expanding.

The delivery tube may be sized to receive a microcatheter so that a thrombectomy device can be used at a more distal location or to use a 0.89 mm (0.035") guidewire to assist access

In a further disclosed system, some or all of the inflation lumen(s) can extend along the delivery tube. One or more of the inflation lumen(s) can be in communication with the distal balloon and the angioplasty balloon, positioned to inflate the distal and angioplasty balloon. The inflation lumen(s) can be further in communication with the proximal balloon, positioned to inflate the proximal balloon.

According to the invention, the one or more inflation lumens includes a singular inflation lumen in communication with both the distal balloon and the angioplasty balloon, positioned to inflate both the distal balloon and the angioplasty balloon. The singular inflation lumen can further be in communication with the proximal balloon, positioned to inflate the proximal balloon. The pressure at which balloons are expandable allows multiple balloons to be inflated by a single inflation lumen in a predetermined order. The balloon expandable at a lower pressure can be inflated before the balloon expandable at a higher pressure. According to the invention, the distal balloon is expandable at a lower pressure compared to an angioplasty balloon inflatable by the same inflation lumen. In which case the distal balloon can be inflated first. In an instance including a proximal balloon inflatable by the same inflation lumen as the distal balloon and the angioplasty balloon, the distal balloon can be expandable at a lower pressure than the proximal balloon and the angioplasty balloon.

As an alternative disclosed system which does not comprise a single inflation lumen in communication with both the distal balloon and the angioplasty balloon, the one or more inflation lumens can include a first inflation lumen in communication with the distal balloon and a second inflation lumen in communication with the angioplasty balloon. Each of the first and second inflation lumens can extend along the delivery tube. The first inflation lumen can be positioned to provide a first flow path to inflate the distal balloon. The second inflation lumen can be positioned to provide a second flow path to inflate the angioplasty balloon. The first and second flow paths can be separated such that the distal balloon and angioplasty balloon are each inflatable independent of each other. In instances where the delivery tube includes a proximal balloon thereon, the first inflation lumen or the second inflation lumen can further be in communication with the proximal balloon, positioned to inflate the proximal balloon. The pressure at which balloons are expandable can allow multiple balloons to be inflated by a single inflation lumen in a predetermined order. As an alternative to the proximal balloon being in communication with an inflation lumen common to the distal balloon or angioplasty balloon, the one or more inflation lumens can further include a third inflation lumen in communication with the proximal balloon. The third inflation lumen can be positioned to provide a third flow path to inflate the proximal balloon. The first, second, and third flow paths can be separated such that the distal balloon, angioplasty balloon, and the proximal balloon are each inflatable independent of each other. The third inflation lumen can extend along the delivery tube; alternatively, the third inflation lumen can extend along a separate tube and/or catheter such as a guide catheter in which the delivery tube is slidably translatable.

An example method for treating an intravascular lesion can include one or more of the following steps presented in no particular order. The methods disclosed herein are not part of the claimed subject matter. The method can further include additional steps as appreciated and understood by a person of ordinary skill in the art according to the teachings of this disclosure.

The method can include positioning a delivery tube having a distal balloon, angioplasty balloon, and stent thereon across the lesion. The delivery tube can be positioned such that the distal balloon is in the distal direction in relation to the lesion, the angioplasty balloon crosses the lesion, and the stent crosses the lesion.

The method can include inflating the distal balloon. The distal balloon can be fully inflated before the angioplasty balloon is fully inflated.

The method can include expanding a proximal expandable element positioned in the proximal direction in relation to the lesion. The proximal expandable element can be expanded such that the proximal expandable element circumferentially apposes a blood vessel before the angioplasty balloon is inflated. The proximal expandable element can include a proximal balloon. The proximal expandable element can be disposed on the delivery tube or on a guide catheter through which the delivery tube extends. Where the proximal balloon is disposed on a guide catheter through which the delivery tube is disposed, the proximal balloon can be inflated prior to crossing the lesion to protect the distal vascular bed from becoming blocked should the lesion be ruptured or should a portion of the lesion become dislodged inadvertently. The proximal balloon can also, in its expanded state, support the distal advancement of the delivery tube through the lesion, should the lesion be difficult to cross.

The method can include inflating the angioplasty balloon. The angioplasty balloon can be fully inflated after the distal balloon is fully inflated. The angioplasty balloon can be inflated before the proximal expandable element is expanded to circumferentially appose the blood vessel.

The method can include expanding the stent into the lesion as a result of fully inflating the angioplasty balloon.

The method can include inflating the distal balloon and the angioplasty balloon both via a single inflation lumen. Alternatively, the method can include inflating the distal balloon and the angioplasty balloon through separate inflation lumens.

The method can include positioning the proximal balloon in in the proximal direction in relation to the lesion when the delivery tube is positioned across the lesion.

The method can include inflating the proximal balloon such that the proximal balloon is fully inflated after the distal balloon is fully inflated and before the angioplasty balloon is fully inflated.

The method can include inflating the distal balloon, the angioplasty balloon, and the proximal balloon via a single inflation lumen.

When the proximal expandable element is disposed on a guide catheter, the method can include positioning the guide catheter at the lesion such that the proximal expandable element is in the proximal direction in relation to the lesion. The method can include delivering the delivery tube through a guide catheter (e.g. a guide catheter on which the expandable element and/or proximal balloon is disposed) to the lesion. The method can further include aspirating through a guide catheter (e.g. a guide catheter on which the expandable element and/or proximal balloon is disposed).

The method can include deflating the angioplasty balloon. The angioplasty balloon can be deflated before the distal balloon.

The method can include deflating the distal balloon. The distal balloon can be deflated after the angioplasty balloon.

Another example method for treating an intravascular lesion can include one or more of the following steps presented in no particular order. The method can further include additional steps as appreciated and understood by a person of ordinary skill in the art according to the teachings of this disclosure.

The method can include occluding a blood vessel in a proximal direction in relation to the lesion and in a proximal direction in relation to a bifurcation to a first branch blood vessel and a second branch blood vessel. The first branch blood vessel can be in communication with the Circle of Willis.

The method can include occluding a second branch blood vessel in a distal direction in relation to the lesion and the bifurcation.

The method can include stenting the lesion while the second branch blood vessel distal to the lesion is occluded and the blood vessel proximal to the lesion is occluded.

The method can include aspirating in the vicinity of the lesion after stenting the lesion.

The method can include aspirating in the vicinity of the lesion while the second branch blood vessel distal to the lesion is occluded and the blood vessel proximal to the lesion is occluded.

Another intravascular treatment system disclosed can include a delivery tube, a first and second balloon, and an inflation lumen. The first and second balloons can be disposed on a distal portion of the delivery tube. The inflation lumen includes a singular inflation lumen in communication with both the first and second balloons to inflate both the first and second balloons. The first balloon can be expandable at a lower pressure than the second balloon to control the order of inflation and deflation of the balloons via the inflation lumen. The treatment system can further include a stent circumscribing the delivery tube 110 and positioned between the first and second balloons. The stent can be self-expandable.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIGs. 1A and 1B are an illustration of an intravascular treatment system during sequential treatment steps according to aspects of the present invention;
FIG. 1C illustrates a cross-section of the intravascular treatment system as indicated in FIG. 1B according to aspects of the present invention;
FIGs. 2A through 2C illustrate variations of a cross-section of a delivery tube of an intravascular treatment system as indicated in FIG. 1B according to aspects of the present invention;
FIGs. 3A through 3J are a sequence of illustrations depicting steps of treatment of an intravascular lesion using the treatment system illustrated in FIG. 1 according to aspects of the present invention;
FIG. 4 is an illustration of another intravascular treatment system according to aspects of the present invention;
FIGs. 5A and 5B illustrate variations of a cross-section of a delivery tube of an intravascular treatment system as indicated in FIG. 4 according to aspects of the present invention;
FIGs. 6A through 6H are a sequence of illustrations depicting steps of treatment of an intravascular lesion using the treatment system illustrated in FIG. 4 according to aspects of the present invention;
FIGs. 7A and 7B are a sequence of illustration depicting alternative steps of treatment of an intravascular lesion using the treatment system illustrated in FIG. 4 according to aspects of the present invention;
FIGs. 8A through 8E are a sequence of illustrations depicting steps of treatment of an intravascular lesion at a bifurcation according to aspects of the present invention;
FIGs. 9A through 9D are a sequence of illustrations depicting steps of treatment utilizing a porous occlusion element according to aspects of the present invention;
FIG. 10 is a flow diagram outlining steps for treating an intravascular lesion using the system according to aspects of the present invention; and
FIG. 11 is a flow diagram outlining steps for treating an intravascular lesion at a bifurcation using the system according to aspects of the present invention.

### DETAILED DESCRIPTION

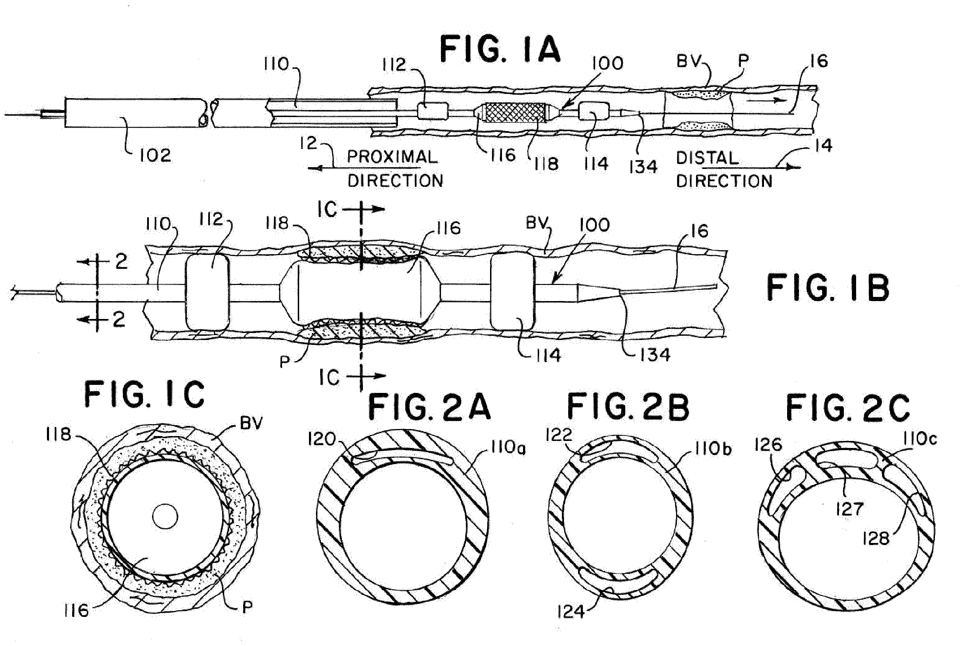

FIGs. 1A through 1C are illustrations of an intravascular treatment system 100 during treatment of a lesion P in a blood vessel BV. The treatment system 100 can include a delivery tube 110, a distal balloon 114, an angioplasty balloon 116, a proximal occlusion device 112, and a stent 118. The system 100 can be sized to traverse vasculature to the lesion P. FIG. 1A illustrates the system 100 in a collapsed delivery configuration being delivered through a catheter 102 to a lesion P (plaque) in a blood vessel BV. FIG. 1B illustrates the system 100 with the proximal occlusion device 112, distal balloon 114, angioplasty balloon 116, and stent 118 expanded. The proximal occlusion device 112 and distal balloon 114 isolate the treatment area around the lesion P. FIG. 1C illustrates a cross section of the system 100 through the angioplasty balloon 116 and stent 118 as indicated in FIG. 1B.

The delivery tube 110 can be sufficiently long such that a proximal end of the delivery tube 110 can be positioned outside the patient when the treatment system 100 is positioned at the lesion P as illustrated in FIG. 1B. Configured as such, a user (e.g. physician) can manipulate a proximal end of the delivery tube 110 to expand and otherwise manipulate portions of the system 100 illustrated in FIGs. 1A through 1C. The system 100 can be delivered over a guide wire 16.

The distal balloon 114 can be disposed on the delivery tube 110 near a distal end 134 of the delivery tube. The angioplasty balloon 116 can be disposed on the delivery tube 110 in the proximal direction 12 in relation to the distal balloon 114. The stent 118 can be disposed over the angioplasty balloon 116. The proximal occlusion element 112 can be disposed on the delivery tube 110 in the proximal direction 12 in relation to the angioplasty balloon 116.

The proximal occlusion element 112 can include a balloon. Alternatively, the proximal occlusion element can include a self-expandable, mechanically expandable, and/or otherwise expandable occlusion element.

The proximal occlusion element 112, distal balloon 114, and angioplasty balloon 116 are configured to expand to appose vascular walls in a blood vessel BV as illustrated in FIGs. 1B and 1C. The distal balloon 114 and proximal occlusion element 112 prevent fragments from the lesion P from migrating away from the lesion P. The angioplasty balloon 116 presses the stent 118 into plaque of the lesion P and can be further inflated to expand the circumference of the blood vessel BV.

As a variation on the treatment system 100 illustrated in FIGs. 1A through 1C, the system can include two balloons inflatable by a single inflation lumen. In one example, the proximal occlusion element 112 and distal balloon 114 can be inflated by the single inflation lumen. In this case, the system 100 need not include an angioplasty balloon. In this example, the stent 118 can be self-expandable and the system 100 can include a sheath covering the stent 118 and retractable to allow the stent to expand. In another example, the angioplasty balloon 116 and the distal balloon 114 can be inflated by a single inflation lumen. In another example, the proximal expandable element 112 and the angioplasty balloon 116 can be inflated by a single inflation lumen.

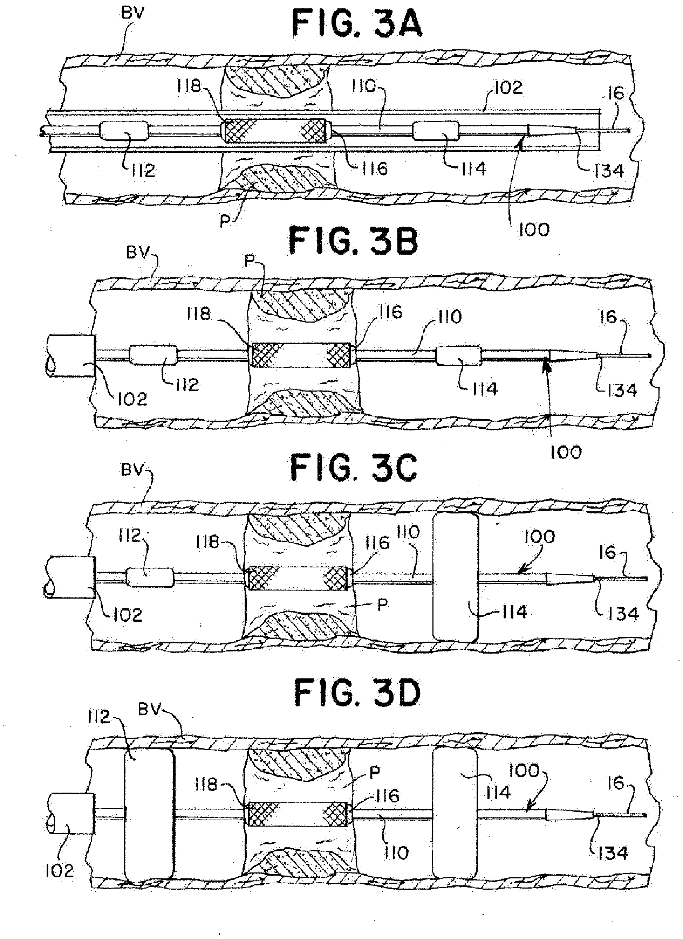

FIGs. 2A through 2C illustrate variations of a cross-section of the delivery tube 110 of the intravascular treatment system 100 as indicated in FIG. 1B. Alternative delivery tube lumen configurations 110a, 110b, 110c illustrated in FIGs. 2A through 2C can be used in the delivery tube 110 illustrated in FIGs. 1A through 1C and in use in FIGs. 3A through 3J.

Referring collectively to FIGs. 1A through 1C and 2A through 2C, the delivery tube 110 can include one or more inflation lumens 120, 122, 124, 126, 128, 220, 222, 224, 224, at least some of which are in communication with the distal balloon 114 and the angioplasty balloon 116, 216, 316. The inflation lumens in communication with the distal balloon 114 and angioplasty balloon extend along the delivery tube 110 and are positioned to inflate the distal balloon 114 and the angioplasty balloon 116. In examples where the proximal occlusion element 112 includes a balloon, at least one of the inflation lumens 120, 122, 124, 126, 127, 128 can further be in communication with the proximal balloon and can be positioned to inflate the proximal balloon 112. Inflation lumens 120, 122, 124, 126, 127, 128 can extend along the system such that they are accessible outside of the patient (e.g. to be connected to a pump) when the system is in positions illustrated in FIGs. 1A through 1C.

FIG. 2A illustrates an example delivery tube 110a having a singular inflation lumen 120. The singular inflation lumen 120 can be in communication with both the distal balloon 114 and the angioplasty balloon 116 to inflate both balloons 114, 116. The distal balloon 114 can be expanded at a lower pressure than the angioplasty balloon 116 such that the distal balloon 114 inflates before the angioplasty balloon 116. To accomplish this, the angioplasty balloon 116 can have an expandable compliant membrane and the distal balloon 114 can have a non-compliant membrane. Alternatively, the angioplasty balloon 116 can have a non-compliant membrane and/or wrap restricted from expanding by the stent 118 when the stent is collapsed as illustrated in FIG. 1A.

When the angioplasty balloon 116 includes a compliant membrane, the compliant membrane can expand as pressure within the angioplasty balloon 116 increases. The non-compliant membrane can expand to one specific size or size range, even as internal pressure within the distal balloon 114 increases, and the distal balloon 114 can require less pressure to inflate to occlude the blood vessel BV compared to the compliant membrane of the angioplasty balloon 116. To reduce the risk of overexpansion of the compliant membrane of the angioplasty balloon 116, the angioplasty balloon 116 can further include and a non-compliant wrap configured to restrict the expanded diameter of the angioplasty balloon 116. As an alternative to using a non-compliant distal balloon 114, the distal balloon 114 can include a complaint membrane that inflates at a lower pressure compared to the compliant membrane of the angioplasty balloon 116. In such a case, to reduce the risk of overexpansion of the compliant membrane of the distal balloon 114, the distal balloon 114 can also include a non-compliant wrap configured to restrict the expanded diameter of the distal balloon 114. Additionally, or alternatively, to reduce the risk of overexpansion of the distal balloon 114, the distal balloon can include a thick walled elastomeric material resistant to expansion compared to the angioplasty balloon's desired expansion. While pressure to fully expand the distal balloon 114 can depend primarily on the material properties of its' balloon membrane, pressure to inflate the angioplasty balloon 116 can depend on the properties of its' balloon membrane in addition to compression force from the stent 118, resistance of the plaque P, and resistance of the blood vessel BV to expansion.

Regardless as to whether the distal balloon 114 is non-compliant or includes a compliant membrane, the angioplasty balloon 116 can be deflated before the distal balloon 114. The membrane of the angioplasty balloon 116 can be sufficiently resilient to press inflation fluid from the angioplasty balloon 116 at a faster rate than inflation fluid is expelled from the distal balloon 114 when inflation pressure is reduced.

When the angioplasty balloon 116 includes a non-compliant membrane, the stent 118 can provide a force to inhibit expansion of the angioplasty balloon 116. The distal balloon 114, being uninhibited by a stent, can thereby inflate at a lower pressure compared to the angioplasty balloon 116. Pressure effective to inflate the angioplasty balloon 116 can be determined based on mechanical properties of the stent 118 which cause the stent 118 stent to be resistant to expansion.

In examples where the proximal occlusion element 112 includes a balloon, the proximal balloon 112 can be expanded via the singular inflation lumen 120 illustrated in FIG. 2A. The proximal balloon 112 can be configured to inflate before the angioplasty balloon 116. The proximal balloon can include a non-compliant membrane that is inflatable to block the blood vessel BV at a lower pressure than the compliant membrane of the angioplasty balloon 116. Alternatively, the proximal balloon 112 can include a compliant membrane that is more compliant than the compliant membrane of the angioplasty balloon 116. In which case, to reduce the risk of overexpansion of the proximal balloon 112, the proximal balloon 112 can further include a non-compliant wrap configured to restrict the expanded diameter of the proximal balloon 112. Regardless as to whether the proximal balloon 112 is non-complaint or includes a compliant membrane, the angioplasty balloon 116 can be deflated before the distal balloon 114. The membrane of the angioplasty balloon 116 can be sufficiently resilient to press inflation fluid from the angioplasty balloon 116 at a faster rate than inflation fluid is expelled from the proximal balloon 112 when inflation pressure is reduced.

In examples where the proximal occlusion element 112 includes a balloon, the proximal balloon 112 can further be configured to inflate before, after, or simultaneously with the distal balloon 114. For instance, the distal balloon 114 can be inflated at a lower pressure than both the angioplasty balloon 116 and the proximal balloon 112 to allow the distal balloon 114 to inflate before both the proximal balloon 112 and the angioplasty balloon 116. To accomplish this, the distal balloon can be non-compliant, and both the proximal and angioplasty balloons 112, 116 can be compliant with a non-compliant wrap. Alternatively, the distal balloon 114 can include a compliant membrane that is configured to expand at a lower pressure than both the proximal balloon 112 and the angioplasty balloon 116.

In some treatments it can be advantageous to inflate the distal balloon 114 first to block blood flow downstream of the lesion P, inflate the proximal balloon 112 second to block blood flow to the lesion P, and inflate the angioplasty balloon 116 third, to expand the stent 118. The distal balloon 114 can anchor the delivery tube 110 in place and block the blood vessel BV while still providing access for other treatment devices to reach the lesion P from the proximal direction 12.

In some treatments it can be advantageous to expand the proximal occlusion element 112 (e.g. inflate a balloon of the proximal occlusion element 112) before inflating the distal balloon 114 and angioplasty balloon 116 to block blood flow to the lesion P. Inflating the proximal balloon 112 first can be particularly advantageous when the angioplasty balloon 116 and/or distal balloon 114 are translatable to cross the lesion P after the proximal occlusion element 112 is expanded to thereby inhibit fragments dislodged during crossing of the lesion P from being carried elsewhere in the vasculature by blood flow.

In another example, the proximal balloon, distal balloon and the angioplasty balloon include non-compliant wraps such that higher pressures can be used to compress the lesion. In yet another example, the proximal balloon, distal balloon and the angioplasty balloon can include a non-compliant balloon so that higher pressures can be used to compress plaque and wherein the sequence of expansion of each balloon is controlled by using compliant wraps, each compliant wrap being configured to release each of the non-compliant balloons in a predetermined sequence as discussed above.

FIG. 2B illustrates an example delivery tube 110b having two inflation lumens 122, 124. The balloons on separate lumens can be inflated and deflated in a desired order and at a desired pressure independent from each other. The two lumens 122, 124 can be isolated from each other along the body of the delivery tube 110b. For instance, a first lumen 122 can be positioned and otherwise configured to inflate the distal balloon 114 and a second lumen 124 can be positioned and otherwise configured to inflate the angioplasty balloon 116. If the proximal occlusion element 112 includes a balloon, two of the three balloons can be inflated by one of the two lumens 122, 124 and the third balloon can be inflated by the other of the two lumens 122, 124. Balloons inflatable by the same inflation lumen can be configured to inflate and deflate in a predetermined order as described in relation to FIG. 2A.

FIG. 2C illustrates an example delivery tube 110c having three inflation lumens 126, 127, 128. If the proximal occlusion element 112 includes a balloon, each balloon 112, 114, 116 can be inflatable by a respective individual lumen 126, 127, 128.

FIGs. 3A through 3J are a sequence of illustrations depicting steps of treatment of an intravascular lesion P using the treatment system 100 illustrated in FIG. 1.

FIG. 3A illustrates the delivery tube 110 positioned across the lesion P within a catheter 102. To reach the position illustrated in FIG. 3A, first a guide wire 16 can be extended through vasculature of the patient and positioned across the lesion P, then the catheter 102 and delivery tube 110 can be translated in the distal direction 14 over the guide wire 16 to the illustrated position.

FIG. 3B illustrates the catheter 102 retracted in the proximal direction 12 to unsheathe a distal portion of the delivery tube 110 including the distal balloon 114, angioplasty balloon 116, stent 118, and proximal occlusion element 112.

FIG. 3C illustrates the distal balloon 114 being inflated to circumferentially appose walls of the blood vessel BV before the angioplasty balloon 116 is inflated or the proximal occlusion element 112 is expanded.

FIG. 3D illustrates the proximal occlusion element 112 expanded to appose walls of the blood vessel BV after the distal balloon 114 is inflated and before the angioplasty balloon 116 is inflated.

The system 100 can additionally, or alternatively be configured such that the proximal occluding element 112 is expandable at the same time or prior to the distal balloon 114. In some examples, the balloon of the proximal occluding element 112 can be expanded at same pressure as distal balloon 114 or the angioplasty balloon 116. Preferably, the proximal and distal balloons are both compliant and inflated at the same pressure so that a compliant (elastomeric) balloon can be used for each such that each balloon has a low profile and does not require wrapping (e.g. with the stent to restrict expansion) like a non-compliant angioplasty balloon 116.

In another example, all balloons 112, 114, 116 can be elastomeric. The balloon 116 used to expand the stent 118 can be made using a thicker or stiffer material such that the proximal and distal balloons 112, 114 expand first (due to thinner wall and thus lower inflation pressure) and when the stent balloon 116 expands, the proximal and distal balloons 112, 114 begin to roll out longitudinally.

In another example, a composite or hybrid balloon can be used. The composite balloon can be constructed of an elastomeric material with non-compliant threads or braid that prevents the balloon from expanding past a certain diameter. The hybrid balloon can be constructed of an inner non-compliant balloon with an elastomeric outer sleeve that holds the non-compliant inner balloon in a compressed configuration for low profile delivery.

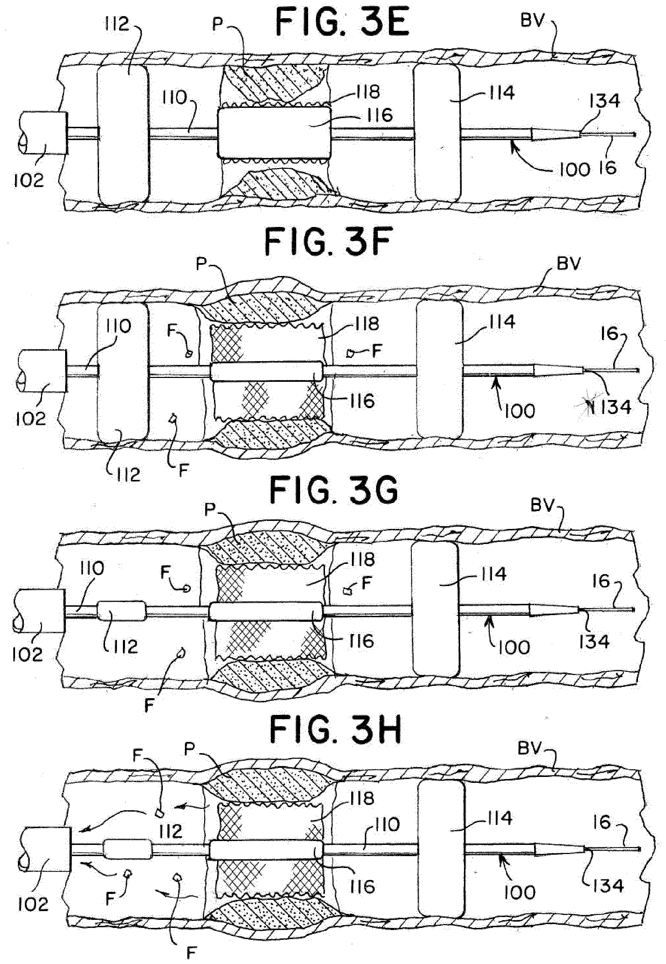

FIG. 3E illustrates the angioplasty balloon 116 and stent 118 expanded. Inflation of the angioplasty balloon 116 can press the stent 118 into the lesion P. Inflation of the angioplasty balloon 116 can additionally expand the blood vessel.

FIG. 3F illustrates the angioplasty balloon 116 deflated while the distal balloon 114 and proximal occlusion element 112 remain expanded to appose walls of the blood vessel BV. The stent 118 can remain expanded upon deflation of the angioplasty balloon 116. Inflation and deflation of the angioplasty balloon 116 can dislodge fragments F from the lesion P. The fragments F can be contained between the expanded distal balloon 114 and proximal occlusion element 112.

FIG. 3G illustrates the proximal occlusion element 112 being collapsed following deflation of the angioplasty balloon 116. The distal balloon 114 can remain expanded to inhibit the fragments F from flowing downstream from the lesion P. Collapsing of the proximal element 112 can allow for additional treatments to be delivered to the lesion P.

FIG. 3H illustrates aspiration through a lumen of the catheter 102 while the distal balloon 114 remains expanded in the blood vessel BV. The lumen of the catheter 102 and the delivery tube 110 can be dimensioned to provide passage for fragments to enter the lumen of the catheter 102 while the delivery tube 110 is extended therethrough. In some examples, the same catheter 102 can be used to deliver the system 100 across the lesion P as illustrated in FIG. 3A and aspirate as illustrated in FIG. 3H. Alternatively, in some applications, it can be advantageous to use two different catheters for the crossing step illustrated in FIG. 3A and the aspiration step illustrated in FIG. 3H. For instance, a small diameter catheter may be better suited to cross the lesion P to minimally disturb the lesion P, and a large mouth catheter may be better suited to capture larger fragments F dislodged from the lesion P.

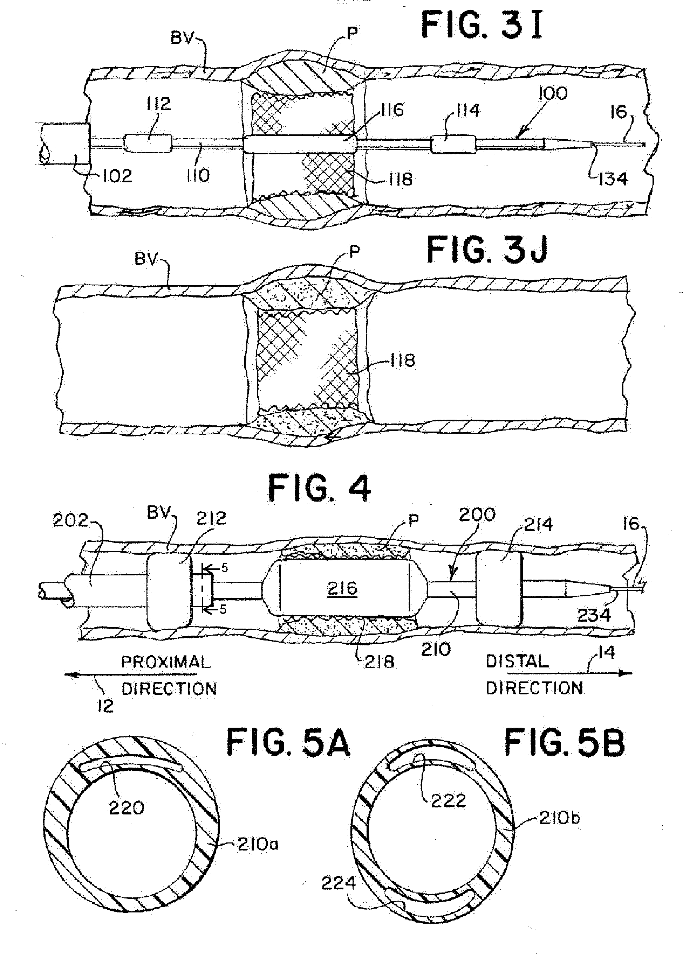

FIG. 3I illustrates the distal balloon 114 deflated following aspiration of the fragments F.

FIG. 3J illustrates the delivery tube 110 retracted from the treatment site with the stent 118 remaining in position supporting the lesion P.

FIG. 4 is an illustration of another intravascular treatment system 200 including a catheter 202 including a proximal occluding element 212 thereon, the system 200 further including a delivery tube 210 including a distal balloon 214, an angioplasty balloon 216, and a stent 218 thereon.

FIGs. 5A and 5B illustrate variations of a cross-section of the delivery tube 210 of the intravascular treatment system 200 as indicated in FIG. 4.

FIG. 5A illustrates a delivery tube 210a having a singular inflation lumen 220 sized, positioned and otherwise configured to inflate both the distal balloon 214 and the angioplasty balloon 216. The distal balloon 214, angioplasty balloon 216, and single inflation lumen 220 can be configured similar to as described in relation to FIG. 2A. The distal balloon 214 can be configured to inflate before the angioplasty balloon 216 via the singular inflation lumen 220. The distal balloon 214 can be configured to deflate after the angioplasty balloon 216 via the singular inflation lumen 220.

FIG. 5B illustrates a delivery tube 210b having two inflation lumens 222, 224. The distal balloon 214 and angioplasty balloon 216 can be separately inflatable via a respective inflation lumen 222, 224.

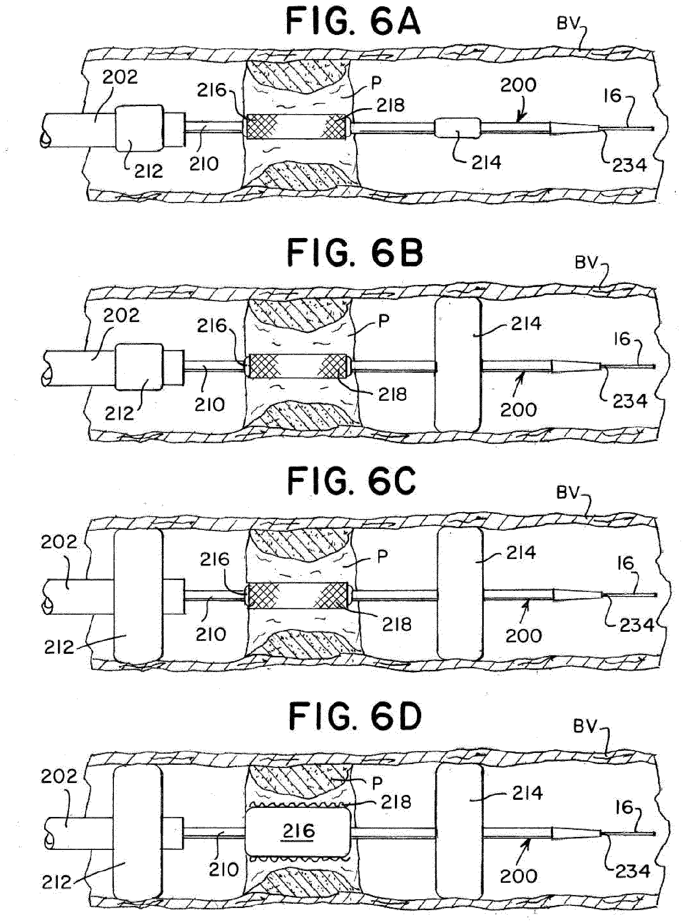

FIGs. 6A through 6H are a sequence of illustrations depicting steps of treatment of an intravascular lesion P using the treatment system 200 illustrated in FIG. 4.

FIG. 6A illustrates the delivery tube 210 extended across the lesion such that the distal balloon 214 is positioned in the distal direction 14 in relation to the lesion P and the stent 218 and angioplasty balloon 216 are positioned through or across the lesion P. The catheter 202 is positioned such that the proximal occlusion element 212 is positioned in the proximal direction 12 in relation to the lesion P. To deliver the system 200 to the position illustrated, first a guide wire 16 can be extended through vasculature of the patient and through the lesion P, then the catheter 202 and delivery tube 210 can be translated distally 14 over the guide wire 16. In some examples, the lumen of the catheter 202 can be sized such that the distal portion of the delivery tube 210 including the collapsed stent 218, angioplasty balloon 216, and distal balloon 214 can fit within the catheter 202. In some treatments it can be advantageous to position the distal balloon 214, stent 218, and angioplasty balloon 216 within the catheter 202 while the delivery tube 210 is translated over the guide wire 16 to the lesion P, then extending the delivery tube 210 out of the catheter 202 to the position illustrated in FIG. 6A.

FIG. 6B illustrates the distal balloon 214 expanded to appose walls of the blood vessel BV prior to the expansion of the angioplasty balloon 216 or proximal occlusion element 212.

FIG. 6C illustrates the proximal occlusion element 212 expanded to appose walls of the blood vessel BV while the distal balloon 214 remains expanded and before the angioplasty balloon 216 is expanded. Configured as such, the blood vessel BV is isolated in the area of the lesion P. The distal end of the catheter 202 is positioned in the distal direction 14 in relation to the expanded proximal occlusion element 212 and the proximal direction 12 in relation to the expanded distal balloon 214. A lumen of the catheter 202 can be sized to allow treatments to be delivered to the isolated lesion P.

FIG. 6D illustrates the angioplasty balloon 216 and stent 218 expanding into the lesion P. The angioplasty balloon 216 can expand the stent 218. In some treatments, the angioplasty balloon 216 can expand the blood vessel BV.

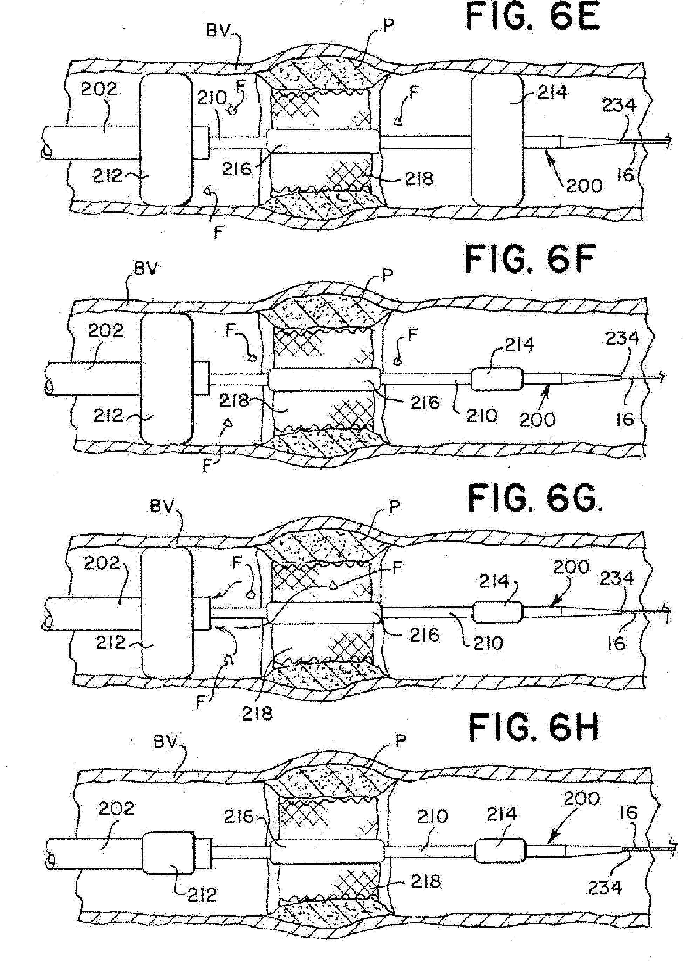

FIG. 6E illustrates the angioplasty balloon 216 deflated. Inflation and deflation of the angioplasty balloon 216 can dislodge fragments F of the lesion P.

FIG. 6F illustrates the distal balloon 214 deflated while the proximal occlusion element 212 remains expanded. The proximal occlusion element 212 can include a fluid impermeable membrane that is effective to inhibit blood flow through the blood vessel BV when the proximal occlusion element 212 is expanded to appose walls of the blood vessel BV. For instance, the proximal occlusion element 212 can include a balloon and/or other expandable structure with a fluid impermeable membrane attached thereto. Deflation of the distal balloon 214 prior to aspiration can reduce risk of blood vessel collapse when both the distal balloon 214 and proximal occlusion element 212 are effective to arrest blood flow.

FIG. 6G illustrates the fragments F being aspirated into the lumen of the catheter 202.

FIG. 6H illustrates the proximal occlusion element 212 collapsed for extraction from the treatment site. The stent 218 can thereafter be left in position across the lesion P similar to as the stent 118 illustrated in FIG. 3J.

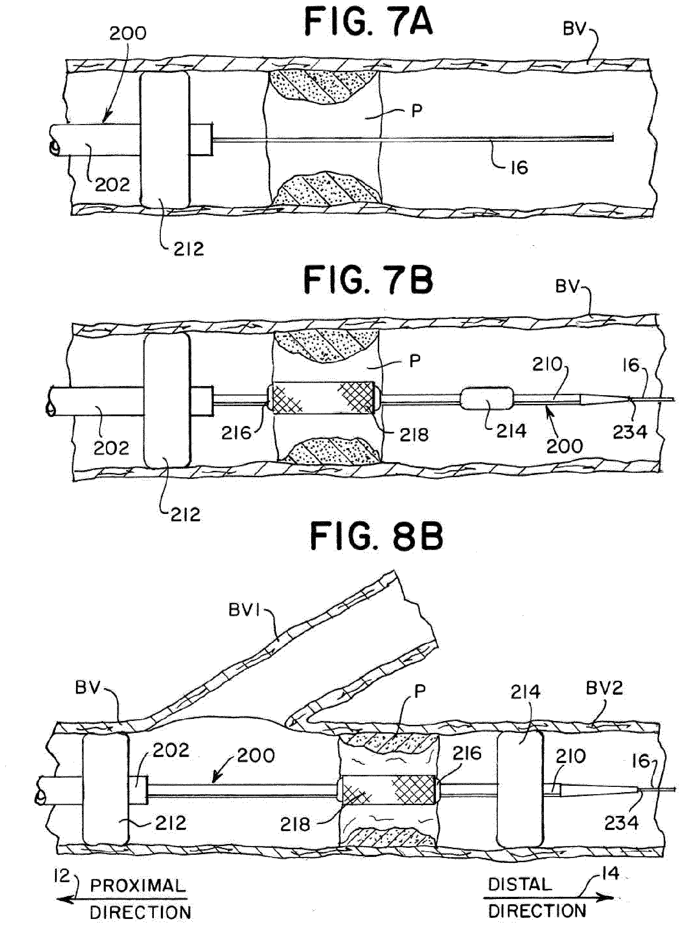

FIGs. 7A and 7B are a sequence of illustration depicting alternative steps of treatment of an intravascular lesion using the treatment system 200 illustrated in FIG. 4.

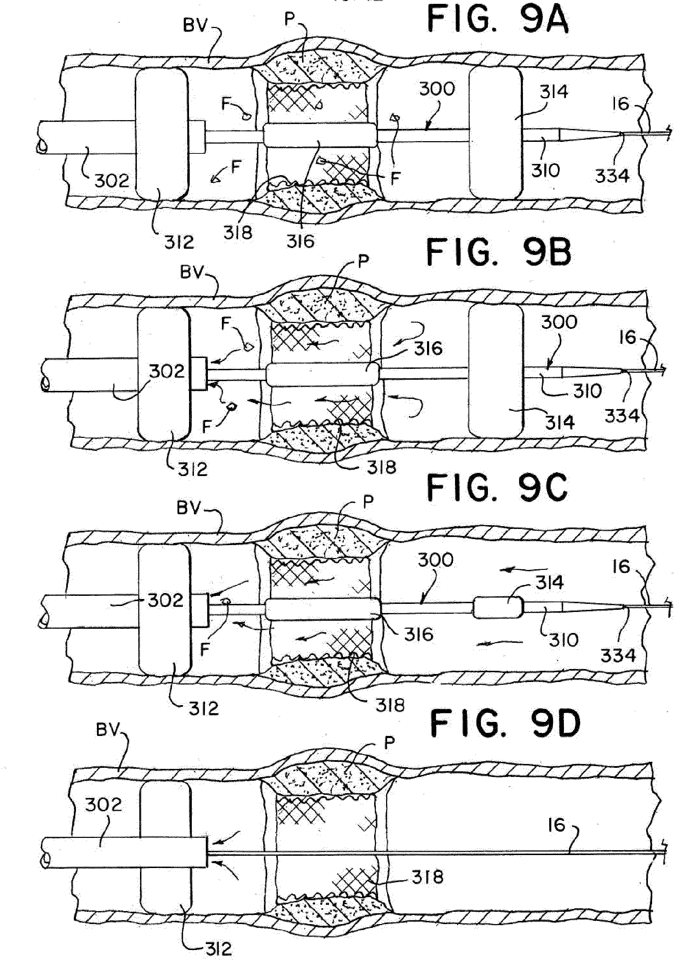

FIG. 7A illustrates the catheter 202 being positioned in the proximal direction 12 in relation to the lesion P and the proximal occlusion element 212 expanded to appose walls of the blood vessel BV in the proximal direction in relation to the lesion. The delivery tube 210 is not yet delivered across the lesion P. The proximal occlusion element 212 can include a fluid impermeable membrane effective to arrest blood flow through the blood vessel BV. The expanded proximal occlusion element 212 can thereby inhibit any fragments dislodged when the delivery tube 210 crosses the lesion P from flowing downstream of the lesion P. Alternatively, the proximal occlusion element 212 can be permeable as the proximal occlusion element 312 is illustrated in FIGs. 9A through 9D.

FIG. 7B illustrates the delivery tube 210 crossing the lesion P after the proximal occlusion element 212 is expanded. The delivery tube 210 and catheter 202 lumen can be sized such the translated through at least a distal portion of the catheter 202 to the position illustrated in FIG. 7B. Once positioned across the lesion, the distal balloon 214 can be inflated, resulting in the configuration illustrated in FIG. 6C. The treatment can thereafter proceed as illustrated in FIGs. 6C through 6H and related description. Alternatively, if the proximal occlusion element 212 is permeable, the treatment can thereafter proceed as illustrated in FIGs. 9A through 9D.

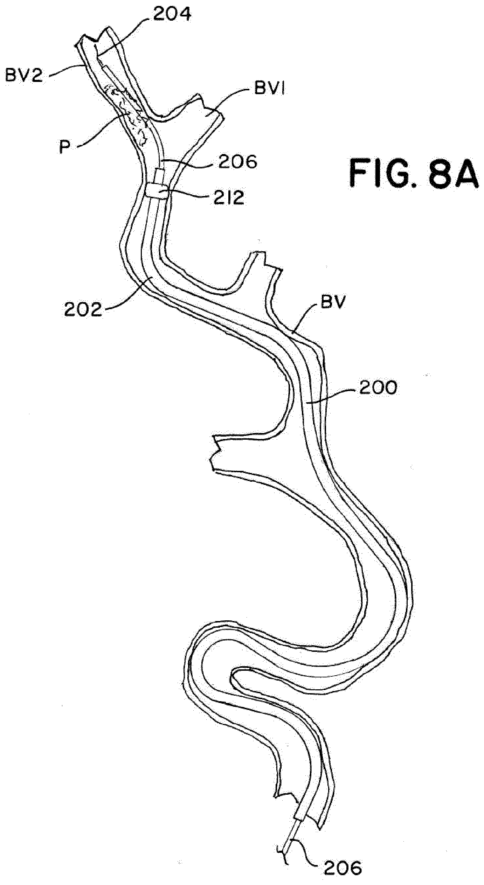

FIGs. 8A through 8E are a sequence of illustrations depicting steps of treatment of an intravascular lesion P at a bifurcation using a system 200 as illustrated in FIG. 4. The bifurcation includes a stem blood vessel BV, a first branch blood vessel BV1, and a second branch blood vessel BV2. The lesion P is positioned in the second branch blood vessel BV2 in a distal direction 14 in relation to the opening to the first branch blood vessel BV1.

FIG. 8A illustrates the delivery tube 210 positioned within a microcatheter or sheath 206 and extended across the lesion P. This illustration depicts an example where the aspiration catheter 202 need not be sized to cross the lesion P. The system 200 is guided over a guidewire 204 to the illustrated position.

FIG. 8B illustrates the delivery tube 210 extended across the lesion P with the distal balloon 214 expanded to occlude the second branch blood vessel BV2 in a distal direction 14 in relation to the lesion P. The angioplasty balloon 216 and stent 218 are positioned across the lesion P. The proximal occlusion element 212 is expanded to appose walls of the stem blood vessel BV in a proximal direction 12 in relation to the bifurcation between the first and second branch blood vessels BV1, BV2. The system 200 can be deployed to the position illustrated in FIG. 8A similar to as illustrated in FIGs. 6A through 6C, FIGs. 7A and 7B, or otherwise deployed as understood by a person of ordinary skill in the art according to the teachings of this disclosure.

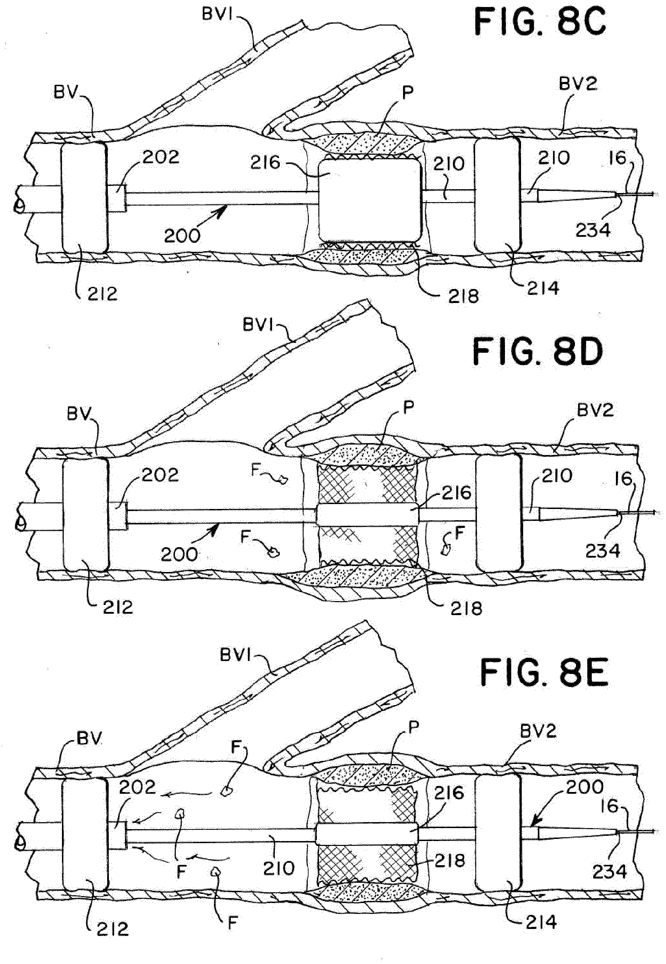

FIG. 8C illustrates the angioplasty balloon 216 inflated to press the stent 218 into the lesion P. The angioplasty balloon 216 can also be inflated to expand the second branch blood vessel BV2 as illustrated. The angioplasty balloon 216 can be inflated while the proximal occlusion element 212 and the distal balloon 214 remain inflated. The proximal occlusion element 212 can be effective to arrest blood flow through the stem vessel BV. The distal occlusion element 214 can be effective to inhibit fragments dislodged from the lesion P from travelling in the distal direction 14 through the second branch vessel BV2.

FIG. 8D illustrates the angioplasty balloon 216 deflated while the stent 218 remains expanded against the lesion P. The fragments F are inhibited from flowing from the lesion site P due to the expansion of the proximal occlusion element 212 and distal balloon 214.

FIG. 8E illustrates aspiration into the lumen of the catheter 202. The lumen of the catheter 202 can be sized, positioned, and otherwise configured to allow passage of the fragments F into the lumen of the catheter 202 while the delivery tube 210 is in place as illustrated in FIG. 8D.

FIGs. 9A through 9D are a sequence of illustrations depicting steps of treatment utilizing an alternative treatment system 300 including a porous occlusion element 312. The system 300 can include a catheter 302 including a proximal occluding element 212 thereon, the system 300 further including a delivery tube 310 including a distal balloon 314, an angioplasty balloon 316, and a stent 318 thereon. The system 300 can be positioned as illustrated in FIG. 9A similar system 200 as illustrated in FIGs. 6A through 6H or FIGs. 7A and 7B or otherwise deployed as understood by a person of ordinary skill in the art according to the teachings herein.

FIG. 9A illustrates fragments F dislodged from the lesion P confined between the expanded distal balloon 314 and proximal occlusion element 312.

FIG. 9B illustrates aspiration through a lumen of the catheter 302. The lumen of the catheter 302 and the delivery tube 310 can be sized such that when the delivery tube 310 is extended through the microcatheter 302 as illustrated, the lumen can receive the fragments F. Pores in the proximal occlusion element 312 can be sized to permit blood to flow through the proximal occlusion element 312 while the distal balloon 314 arrests blood flow through the blood vessel BV. As fragments F are aspirated into the catheter 302, flow of blood through the proximal occlusion element 312 can permit aspiration in an isolated region of the blood vessel with low risk of blood vessel collapse. The pores of the proximal occlusion element 312 can be sized to inhibit fragments F from passing through the proximal occlusion element 312. In some treatments, the expanded proximal occlusion element 312 can inhibit fragments F from migrating in the proximal direction 12 in relation to the lesion P.

FIG. 9C illustrates the distal balloon 314 collapsed for extraction of the delivery tube 310. The catheter 302 can continue to aspirate as the delivery tube 310 is retracted across the lesion to collect any further fragments F dislodged from the lesion.

FIG. 9D illustrates the delivery tube 310 retracted into the catheter 302. The catheter 302 can continue to aspirate.

As an alternative to the system 300 illustrated in FIGs. 9A through 9D, the distal balloon 314 can be replaced with a porous occlusion element and the porous proximal occlusion element 312 can include a balloon or other fluid-impermeable structure.

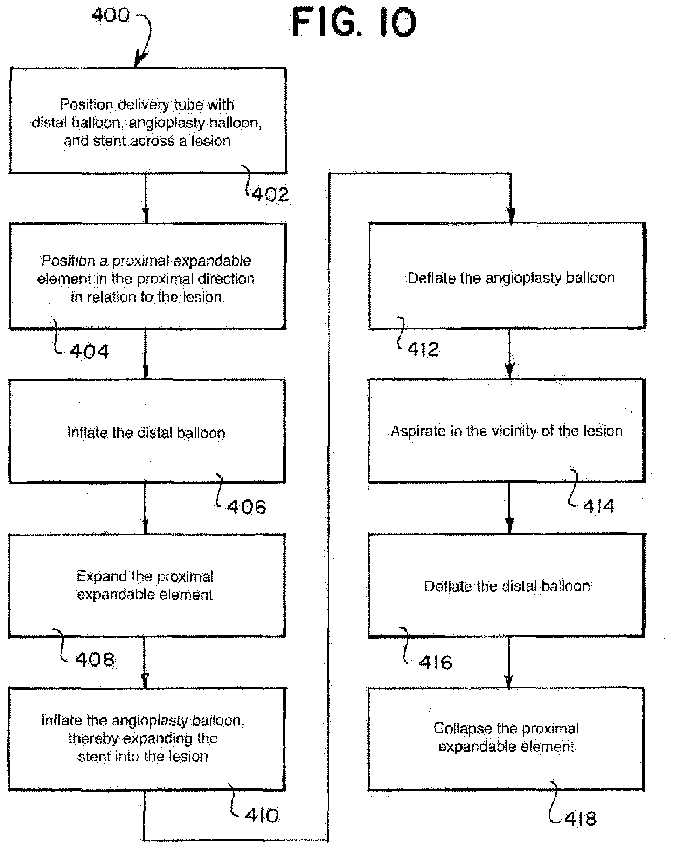

FIG. 10 is a flow diagram of a method 400 for treating an intravascular lesion. In step 402, a delivery tube with a distal balloon, angioplasty balloon, and stent thereon can be placed across a lesion. The delivery tube can be a delivery tube 110, 210, 310 as illustrated and described herein, a variation thereof, or an alternative thereto as appreciated and understood by a person of ordinary skill in the art according to the teaching herein.

In step 404, a proximal expandable element can be positioned in a proximal direction in relation to the lesion. The proximal expandable element can be a proximal occlusion element 112, 212, 312 as illustrated and described herein, a variation thereof, or an alternative thereto as appreciated and understood by a person of ordinary skill in the art according to the teaching herein. The proximal expandable element can be positioned on the delivery tube or can be positioned on a separate device. The proximal expandable element can be fixed a predetermined distance from the distal balloon and/or angioplasty balloon or the proximal expandable element can be slidably translatable in relation to the distal balloon and/or angioplasty balloon.

In step 406, the distal balloon can be inflated. The distal balloon can be inflated through an inflation lumen as described herein or otherwise inflated appreciated and understood by a person of ordinary skill in the art according to the teaching herein.

In step 408, the proximal expandable element can be expanded. The proximal expandable element can be inflated, unsheathed to self-expand, or otherwise expanded as appreciated and understood by a person of ordinary skill in the art according to the teaching herein. Step 408 can be performed before or after step 406 according to the needs of the treatment.

In step 410, the angioplasty balloon can be inflated, thereby expanding the stent into the lesion. The angioplasty balloon can be an angioplasty balloon 116, 216, 316 as illustrated and described herein, a variation thereof, or an alternative thereto as appreciated and understood by a person of ordinary skill in the art according to the teaching herein. The stent can be a stent 118, 218, 318 as illustrated and described herein, a variation thereof, or an alternative thereto as appreciated and understood by a person of ordinary skill in the art according to the teaching herein. The angioplasty balloon can be inflated while both the distal balloon and the proximal expandable element remain expanded. Inflation of the angioplasty balloon can additionally widen the blood vessel.

In step 412, the angioplasty balloon can be deflated. The angioplasty balloon can be deflated while the distal balloon and the proximal expandable element remain expanded.

In step 414, the blood vessel can be aspirated in the vicinity of the lesion. Aspiration can occur while one or both of the proximal expandable element and distal balloon remains expanded.

In step 416, the distal balloon can be deflated. The distal balloon can be deflated via an inflation lumen as illustrated and described herein or otherwise deflated as understood by a person of ordinary skill in the art according to the teaching herein. Step 416 can be performed before or after step 412 according to the needs of the treatment.

In step 418, the proximal expandable element can be collapsed. The proximal expandable element can be deflated, retracted into a sheath, or otherwise collapsed as understood by a person of ordinary skill in the art according to the teaching herein. Step 418 can be performed before or after steps 412 and 416 according to the needs of the treatment.

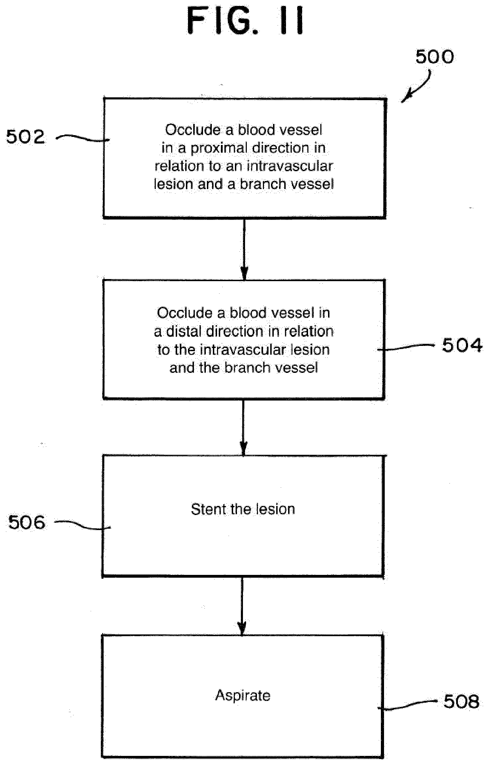

FIG. 11 is a flow diagram of a method 500 for treating an intravascular lesion at a bifurcation. In step 502, a blood vessel can be occluded in a proximal direction in relation to a bifurcation between two branch vessels and a lesion positioned in one of the two branch vessels.

In step 504, one of the two branch vessels can be occluded in a distal direction in relation to the lesion and the bifurcation.

In step 506, a stent can be expanded into the lesion. The stent can be positioned and expanded while vasculature remains occluded as in steps 502 and 504.

In step 508, vasculature can be aspirated in the vicinity of the lesion. The lesion can be aspirated while the vasculature remains occluded as in steps 502 and 504. The non-occluded branch vessel can provide a path for backflow of blood during aspiration.

The descriptions contained herein examples of embodiments of the invention and are not intended in any way to limit the scope of the invention. As described herein, the invention contemplates many variations and modifications of the intravascular treatment system, including alternative materials, alternative device structures, etc. The scope of the invention is therefore defined by the claims.

## Claims

1. An intravascular treatment system (100, 200, 300) comprising:
a delivery tube (110, 210, 310) sized to traverse vasculature;
a distal balloon (114, 214, 314) disposed on the delivery tube (110, 210, 310) and approximate a distal end (134, 234, 334) of the delivery tube (110, 210, 310);
an angioplasty balloon (116, 216, 316) disposed on the delivery tube (110 210, 310) in the proximal direction in relation to the distal balloon (114, 214, 316);
a balloon expandable stent (118, 218, 318) disposed over the angioplasty balloon (116, 216, 316); and
one or more inflation lumens in communication with the distal balloon (114, 214, 314) and the angioplasty balloon and extending along the delivery tube such that the one or more inflation lumens are positioned to inflate the distal balloon (114, 214, 314) and the angioplasty balloon;
wherein the one or more inflation lumens is a singular inflation lumen (120, 220) in communication with both the distal balloon (114, 214, 314) and the angioplasty balloon (116, 216), and
wherein the distal balloon (114, 214, 314) is expandable at a lower pressure than the angioplasty balloon (116, 216).

2. The intravascular treatment system (100, 200, 300) of claim 1,
wherein the angioplasty balloon (116, 216) comprises an expandable non-compliant wrap configured to expand the balloon expandable stent (118, 218).

3. The intravascular treatment system (100, 200, 300) of claim 1 or claim 2, further comprising:
a proximal balloon (112, 212) in the proximal direction in relation to the angioplasty balloon (116, 216),
wherein the one or more inflation lumens is further in communication with the proximal balloon and positioned to inflate the proximal balloon; and
an aspiration catheter disposed over the distal balloon (114, 214, 314), angioplasty balloon (116, 216, 316), stent, and proximal balloon.

4. The intravascular treatment system (100, 200, 300) of claim 3,
wherein the one or more inflation lumens is a singular inflation lumen in communication with the distal balloon (114, 214, 314), the angioplasty balloon (116, 216, 316), and the proximal balloon, and
wherein the distal balloon (114, 214, 314) is expandable at a lower pressure than the angioplasty balloon (116, 216, 316).

5. The intravascular treatment system (100, 200, 300) of claim 1 or claim 2, further comprising:
a proximal balloon disposed on the delivery tube in the proximal direction in relation to the angioplasty balloon,
wherein the one or more inflation lumens further comprises a second inflation lumen in communication with the proximal balloon and extending along the delivery tube such that the second inflation lumen is positioned to provide a second flow path to inflate the proximal balloon, and
wherein the first and second flow paths are separated such that the distal balloon (114, 214, 314) and the angioplasty balloon (116, 216, 316), and the proximal balloon are each inflatable independent of each other.

6. The intravascular treatment system (100, 200, 300) of any one of claims 1 to 5, further comprising:
a catheter (102, 202, 302) comprising an expandable element thereon, the catheter sized to traverse vascular and sized to allow the delivery tube (110, 210, 310), distal balloon (114, 214, 314), angioplasty balloon (116, 216, 316), and stent to traverse therethrough.

7. The intravascular treatment system (100, 200, 300) of claim6, wherein the expandable element is porous.

## Patentansprüche

1. Intravaskuläres Behandlungssystem (100, 200, 300), umfassend:
einen zum Durchgang durch das Gefäßsystem bemessenen Zuführschlauch (110, 210, 310),
einen distalen Ballon (114, 214, 314), der an dem Zuführschlauch (110, 210, 310) und in der Nähe eines distalen Endes (134, 234, 334) des Zuführschlauchs (110, 210, 310) angeordnet ist,
einen Angioplastie-Ballon (116, 216, 316), der an dem Zuführschlauch (110, 210, 310) in der proximalen Richtung bezüglich des distalen Ballons (114, 214, 314) angeordnet ist,
einen mittels Ballon expandierbaren Stent (118, 218, 318), der über dem Angioplastie-Ballon (116, 216, 316) angeordnet ist, und
ein oder mehrere Inflationslumen in Verbindung mit dem distalen Ballon (114, 214, 314) und dem Angioplastie-Ballon, die sich entlang des Zuführschlauchs erstrecken, so dass das eine oder die mehreren Inflationslumen zum Inflatieren des distalen Ballons (114, 214, 314) und des Angioplastie-Ballons positioniert sind,
wobei das eine oder die mehreren Inflationslumen ein einziges Inflationslumen (120, 220) in Verbindung sowohl mit dem distalen Ballon (114, 214, 314) als auch mit dem Angioplastie-Ballon (116, 216, 316) ist und
wobei der distale Ballon (114, 214, 314) bei einem niedrigeren Druck als der Angioplastie-Ballon (116, 216) expandierbar ist.

2. Intravaskuläres Behandlungssystem (100, 200, 300) nach Anspruch 1,
wobei der Angioplastie-Ballon (116, 216) eine expandierbare, nicht nachgiebige Hülle umfasst, die zum Expandieren des mittels Ballon expandierbaren Stents (118, 218) ausgestaltet ist.

3. Intravaskuläres Behandlungssystem (100, 200, 300) nach Anspruch 1 oder Anspruch 2, ferner umfassend:
einen proximalen Ballon (112, 212) in der proximalen Richtung bezüglich des Angioplastie-Ballons (116, 216),
wobei das eine oder die mehreren Inflationslumen ferner mit dem proximalen Ballon in Verbindung sind und dazu positioniert sind, den proximalen Ballon zu inflatieren, und
einen Aspirationskatheter, der über dem distalen Ballon (114, 214, 314), dem Angioplastie-Ballon (116, 216, 316), dem Stent und dem proximalen Ballon angeordnet ist.

4. Intravaskuläres Behandlungssystem (100, 200, 300) nach Anspruch 3,
wobei das eine oder die mehreren Inflationslumen ein einziges Inflationslumen in Verbindung mit dem distalen Ballon (114, 214, 314), dem Angioplastie-Ballon (116, 216, 316) und dem proximalen Ballon ist und
wobei der distale Ballon (114, 214, 314) bei einem niedrigeren Druck als der Angioplastie-Ballon (116, 216, 316) expandierbar ist.

5. Intravaskuläres Behandlungssystem (100, 200, 300) nach Anspruch 1 oder Anspruch 2, ferner umfassend:
einen proximalen Ballon, der in der proximalen Richtung bezüglich des Angioplastie-Ballons an dem Zuführschlauch angeordnet ist,
wobei das eine oder die mehreren Inflationslumen ferner ein zweites Inflationslumen in Verbindung mit dem proximalen Ballon umfassen, das sich entlang des Zuführschlauchs erstreckt, so dass das zweite Inflationslumen zur Bereitstellung eines zweiten Strömungswegs zum Inflatieren des proximalen Ballons positioniert ist, und
wobei der erste und der zweite Strömungsweg getrennt sind, so dass der distale Ballon (114, 214, 314) und der Angioplastie-Ballon (116, 216, 316) und der proximale Ballon jeweils unabhängig voneinander inflatierbar sind.

6. Intravaskuläres Behandlungssystem (100, 200, 300) nach einem der Ansprüche 1 bis 5, ferner umfassend:
einen Katheter (102, 202, 302), der ein expandierbares Element daran umfasst, wobei der Katheter zum Durchgang durch das Gefäßsystem bemessen sowie so bemessen ist, dass der Zuführschlauch (110, 210, 310), der distale Ballon (114, 214, 314), der Angioplastie-Ballon (116, 216, 316) und der Stent dort hindurchgehen können.

7. Intravaskuläres Behandlungssystem (100, 200, 300) nach Anspruch 6, wobei das expandierbare Element porös ist.

## Revendications

1. Système de traitement intravasculaire (100, 200, 300) comprenant :
un tube de pose (110, 210, 310) dimensionné pour traverser un système vasculaire ;
un ballonnet distal (114, 214, 314) disposé sur le tube de pose (110, 210, 310) et à proximité d'une extrémité distale (134, 234, 334) du tube de pose (110, 210, 310) ;
un ballon d'angioplastie (116, 216, 316) disposé sur le tube de pose (110, 210, 310) dans la direction proximale par rapport au ballonnet distal (114, 214, 316) ;
une endoprothèse expansible par ballonnet (118, 218, 318) disposée sur le ballon d'angioplastie (116, 216, 316) ; et
une ou plusieurs lumières de gonflage en communication avec le ballonnet distal (114, 214, 314) et le ballon d'angioplastie et s'étendant le long du tube de pose de sorte que la ou les lumières de gonflage sont positionnées pour gonfler le ballonnet distal (114, 214, 314) et le ballon d'angioplastie ;
la ou les lumières de gonflage étant une lumière de gonflage (120, 220) unique en communication à la fois avec le ballonnet distal (114, 214, 314) et le ballon d'angioplastie (116, 216), et
le ballonnet distal (114, 214, 314) étant expansible à une plus faible pression que le ballon d'angioplastie (116, 216).

2. Système de traitement intravasculaire (100, 200, 300) selon la revendication 1,
le ballon d'angioplastie (116, 216) comprenant une enveloppe expansible non flexible conçue pour augmenter le volume de l'endoprothèse expansible par ballonnet (118, 218).

3. Système de traitement intravasculaire (100, 200, 300) selon la revendication 1 ou la revendication 2, comprenant en outre :
un ballonnet proximal (112, 212) dans la direction proximale par rapport au ballon d'angioplastie (116, 216),
la ou les lumières de gonflage étant en outre en communication avec le ballonnet proximal et positionnées pour gonfler le ballonnet proximal ; et
un cathéter d'aspiration disposé sur le ballonnet distal (114, 214, 314), le ballon d'angioplastie (116, 216, 316), l'endoprothèse, et le ballonnet proximal.

4. Système de traitement intravasculaire (100, 200, 300) selon la revendication 3,
la ou les lumières de gonflage étant une lumière de gonflage unique en communication avec le ballonnet distal (114, 214, 314), le ballon d'angioplastie (116, 216, 316) et le ballonnet proximal, et
le ballonnet distal (114, 214, 314) étant expansible à une plus faible pression que le ballon d'angioplastie (116, 216, 316).

5. Système de traitement intravasculaire (100, 200, 300) selon la revendication 1 ou la revendication 2, comprenant en outre :
un ballonnet proximal disposé sur le tube de pose dans la direction proximale par rapport au ballon d'angioplastie,
la ou les lumières de gonflage comprenant en outre une deuxième lumière de gonflage en communication avec le ballonnet proximal et s'étendant le long du tube de pose de sorte que la deuxième lumière de gonflage est positionnée afin de fournir un deuxième trajet d'écoulement pour gonfler le ballonnet proximal, et
les premier et deuxième trajets d'écoulement étant séparés de sorte que le ballonnet distal (114, 214, 314) et le ballon d'angioplastie (116, 216, 316), et le ballonnet proximal peuvent chacun être gonflés indépendamment les uns des autres.

6. Système de traitement intravasculaire (100, 200, 300) selon l'une quelconque des revendications 1 à 5, comprenant en outre :
un cathéter (102, 202, 302) comprenant un élément expansible sur celui-ci, le cathéter étant dimensionné pour traverser un système vasculaire et dimensionné pour permettre au tube de pose (110, 210, 310), au ballonnet distal (114, 214, 314), au ballon d'angioplastie (116, 216, 316), et à l'endoprothèse de le traverser.

7. Système de traitement intravasculaire (100, 200, 300) selon la revendication 6, l'élément expansible étant poreux.
